Europäisches Patentamt

European Patent Office

Office européen des brevets

(19)

(11) Publication number: **0 180 125**
A2

(12)

# EUROPEAN PATENT APPLICATION

(21) Application number: **85113327.2**

(22) Date of filing: **21.10.85**

(51) Int. Cl.⁴: **A 61 B 17/06**

(30) Priority: **26.10.84 JP 224058/84**

(43) Date of publication of application: **07.05.86**
**Bulletin 86/19**

(84) Designated Contracting States: **CH DE FR GB IT LI NL**

(71) Applicant: **Karasawa, Yukihiko,**
**No. 606, 2-20-15 Shimouma, Setagaya-Ku Tokyo 154 (JP)**

(72) Inventor: **Karasawa, Yukihiko,**
**No. 606, 2-20-15 Shimouma, Setagaya-Ku Tokyo 154 (JP)**

(74) Representative: **Patentanwälte Viering & Jentschura,**
**Steinsdorfstrasse 6, D-8000 München 22 (DE)**

(54) **A suture needle and its manufacturing processes.**

(57)    A suture needle comprises a suture (3) and a needle body (1) in which a groove (1a) is extended in a substantially overall longitudinal direction of the needle body (1). The suture is inserted into the groove and firmly fixed therein by caulking both sides of the groove. Accordingly, disengagement of the suture from the needle body is entirely prevented. The suture needle which is used for surgical treatment has excellent performance and strength.

A SUTURE NEEDLE AND ITS MANUFACTURING PROCESSES

This invention relates to a suture needle and its manufacturing processes. More particularly, the suture needle is used for medical treatment, in which a suture (thread for sewing up a wound) is previously inserted and fixed within a needle body.

Conventionally, there are knwon two types of suture needles. According to a first type of suture needle, every time a needle is used, a suture is hooked in an aperture of a needle body. According to a second type, the suture is previously fixed in a hole formed at a front part of the needle body at the manufacturing stage. In the first type, every time the suture needle is used, sterilization is required and the suture may be disengaged from the aperture of the needle body, so that it is an old-fashioned type. Today, the second type is prevailing in advanced countries. That is, it is also a disposable type. A typical example of this type is disclosed in the Examined Japanese Patent Publication No. 58-39544 claiming the priority of basic U.S. Patent Application Ser. No. 258159 filed May 21, 1972.

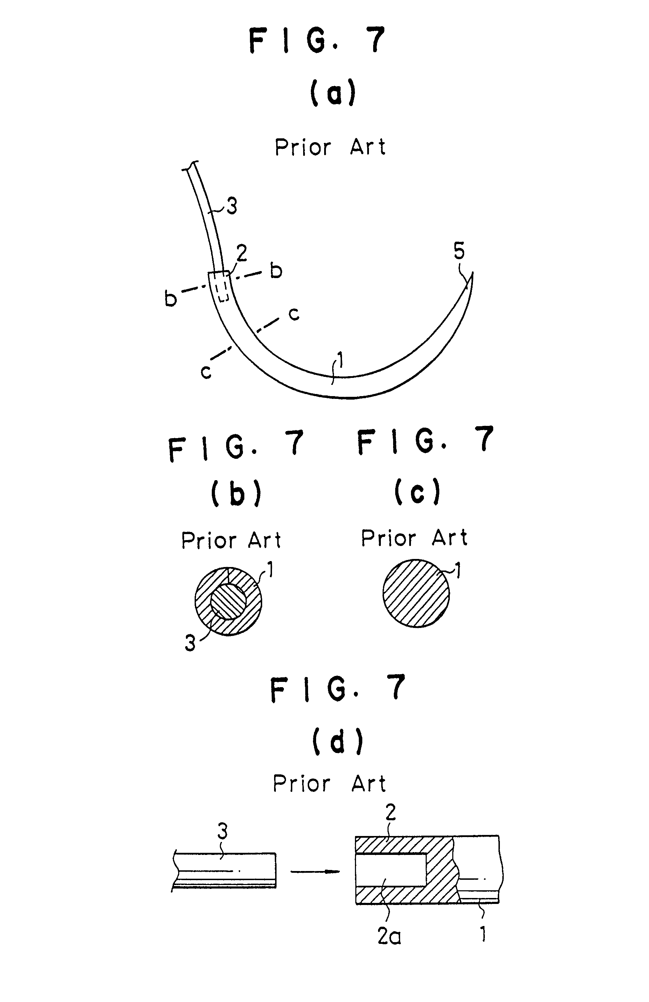

Fig. 7(a) shows a view of a suture needle which is frequently used today.

Numeral 1 is a semicircular needle body made of stainless steel, which comprises a fixing part 2 for fixing a suture 3 at an end of the needle body 1. As shown in 7(d), the fixing part 2 consists of a hole 2a formed in an axial direction of the needle body 1. The suture 3 is inserted into the hole 2a and firmly fixed therein by caulking means.

According to a conventional process for manufacturing needles, a stainless steel thin rod is cut off with a suitable length for a needle body.

Then, the hole 2a is bored by laser processing, drilling or the like. Subsequently, the suture 3 is inserted into the hole 2a manually and fixed therein by caulking means.

The prior art has the following disadvantages and inconveniences.

(1) The depth of the hole 2a for inserting the suture 3 therein is limited in view of boring operation, so that fixation of the suture in the hole 2a is not always complete. Accordingly, disengagement of the suture from the hole may occur easily. If caulking stress is too strong, the needle body 1 may be broken or deformed.

(2) Since the needle body is provided, at one end, with the hole for inserting the suture, the needle body is inclined to be broken in the proximity of the hole.

(3) Since the needle body except for the hole portion is of a medium dense construction, its resilience is relatively low. Accordingly, there grows the danger that a pointed end of the needle body may be broken and retain in a body during sewing operation. Such accident may cause a serious condition.

(4) After the hole has been bored and the suture has been fixed therein, it is no more possible to clean the interior of the hole. Accordingly, it is impossible to wipe away any foreign matter that may remain in the hole during boring operation.

(5) After the suture has been inserted in the hole, sterilization must be carried out, but an inserted part of the suture is inconvenient for a complete sterilization.

(6) The boring operation including laser processing, drilling or the like requires high accuracy, so that it is a cumbersome work. It is very difficult to obtain a bore of a very accurate diameter effective to insert the suture thereinto.

(7) It is also a very cumbersome work even for skilled workers to insert and fix a soft and thin suture into such a minute hole. The daily output of such conventional suture needles is so limited that the production cost is expensive and quality is not always uniform.

It is an object of the invention to provide a suture needle which comprises a suture and a needle body in which a groove is extended in a substantially overall longitudinal direction of the needle body.

More particularly, the suture is inserted into the groove and firmly fixed therein by caulking both sides of the groove. Accordingly, disengagement of the suture from the needle body is entirely prevented.

It is another object of the invention to provide a process for manufacturing a suture needle, comprising the following steps:

preparing an elongated and flattened metal plate having a slightly wider width than that of a needle body;

forming a large number of flattened and segmented patterns each being a needle body, from said elongated and flattened metal plate by means of continuous punch press;

associating a pointed end of each flattened pattern integrally with a side portion of said elongated and flattened metal plate;

forming a groove by bending both sides of each flattened and segmented pattern and inserting said suture into said groove;

fixing said suture in said groove by caulking both sides of said groove under pressure and forming a needle body; and

departing said pointed end integrally associated with said side portion of said elongated and flattened metal plate from said metal body and producing a finished suture needle.

This will now be further described, by way of embodiments, with reference to the accompanying drawings.

Fig. 1 is a section view of a suture needle according to this invention, in which a suture is incorporated in a groove of a needle body.

Fig. 2 is a perspective view of a condition, in which the stuture is about to be inserted into the groove of the needle body in Fig. 1.

Fig. 3 is a section view taken on line III-III of Fig. 1.

Fig. 4 is a perspective view of a condition, in which a flattened pattern of a metal plate is transformed into a grooved pattern.

Fig. 5(a) is a perspective view of a rolled metal plate, from which a large number of flattened patterns are formed by means of punch press.

Fig. 5(b) is a plan view of a flattened metal plate, from which a plurality of flattened patterns are punch-pressed, but a pointed end of each pattern is still integrally associated with the metal plate.

Fig. 5(c) is a perspective view of the suture needle in which the suture is fixed.

Fig. 5(d) is a perspective view of the suture needle which was separated from the metal plate.

Fig. 6(a) is a plan view of another process for manufacturing a suture needle according to the invention.

Fig. 6(b) is a perspective view of a suture needle manufacutred by the process in Fig. 6(a).

Fig. 6(c) is a view of a triangular part of a suture needle formed as a result of grinding.

Fig. 6(d) is a view of a pointed part of a suture needle formed as a result of grinding.

Fig. 6(e) is a perspective view of one example of a finished suture needle.

Fig. 7(a) is a perspective view of a conventional suture needle.

Fig. 7(b) and 7(c) are section views of Fig. 7(a).

Fig. 7(d) is a side view, in which a suture is inserted into a hole of a conventional needle body.

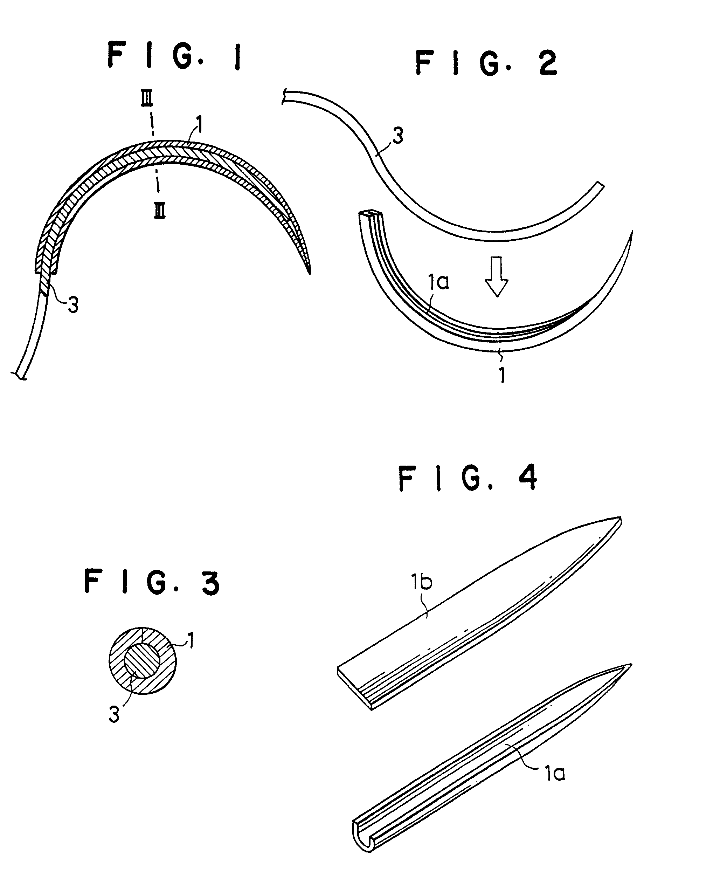

A typical example according to an appended claim 1 of the invention is shown in Figs. 1 to 4.

In Fig. 1, there is shown a needle body 1, in which a groove 1a is extended through a nearly whole length of the needle body 1 and a suture 3 is inserted into the groove 1a.

In Fig. 4, there is shown a flattened plate lb of the needle body material, which is produced by punching press. By bending both sides of the flattened plate lb, the groove la is extended through the nearly whole length of the needle body.  Thus, one suture 3 is inserted wholly into the groove la, and hermetically fixed therein by caulking both sides of the groove la.  As shown in Fig. 3, the section of the caulked needle body 1 is circular, but it may be formed triangular or other preferred shape.

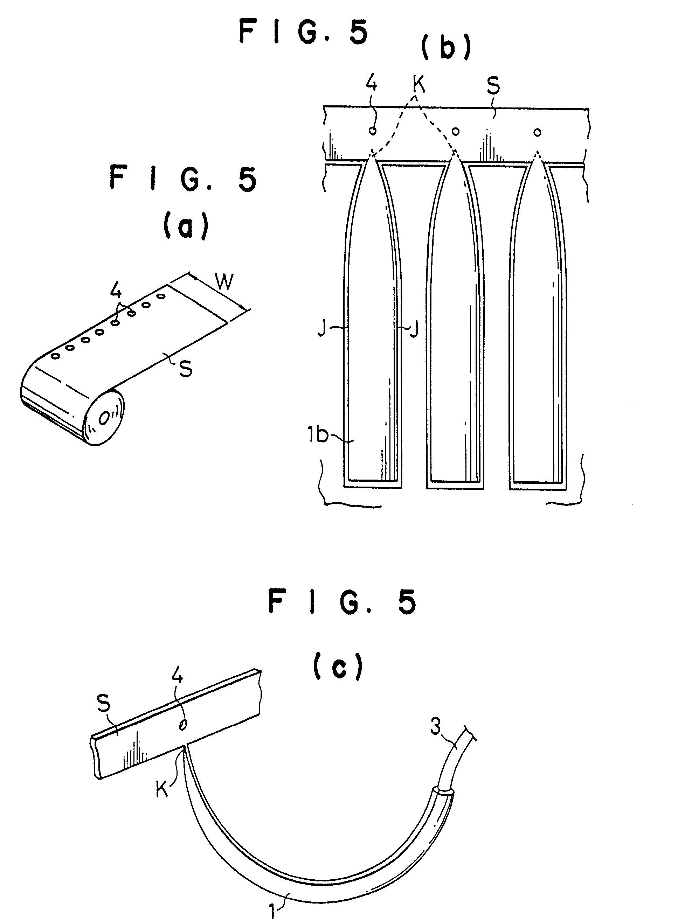

A first embodiment of the process for manufacturing a suture needle according to the invention will be described with reference to Figs. 5(a) to 5(d).  This embodiment corresponds to claim 2 of the invention.

Symbol S is a stainless steel plate having a certain thickness.  Symbol W is a width of the stainless steel plate S which is slightly longer than the whole length of the needle body 1.

As shown in Fig. 5(a), a rolled stainless steel plate S is extended flat and a desired number of pilot openings 4 are formed with a certain space upon a side portion of the stainless steel plate S.

Next to this step, a flattened pattern lb of the needle body 1 must be punch-pressed, so that a pointed end of each pattern lb is directed to each pilot opening 4.  At that time, punch press is carried out only upon a solid line J, i.e. an outer profile of the pattern lb, but an imaginery line K thereof, i.e. a pointed end is remained as it is.  That is, only the pointed end of each pattern lb is integrally associated with the stainless steel plate S.

Next to this step, as shown in Fig. 5(c), both sides of the pattern (flattened plate) lb is bent in a longitudinal direction, thereby the groove 1a is extended through the needle body 1. Then, the needle body 1 is as a whole curved in a nearly semicircular form.

Following this step, the suture 3 is inserted into the whole of the groove 1a, and firmly fixed therein by caulking both sides of the groove 1a, so that the section of the needle body 1 becomes substantially circular.

Subsequently, the imaginary line K of the original pattern 1b is cut off and the needle body 1 is removed from the stainless steel plate S. Thus, there are manufactured continuously a large number of finished suture needles, each of which contains a built-in suture 3.

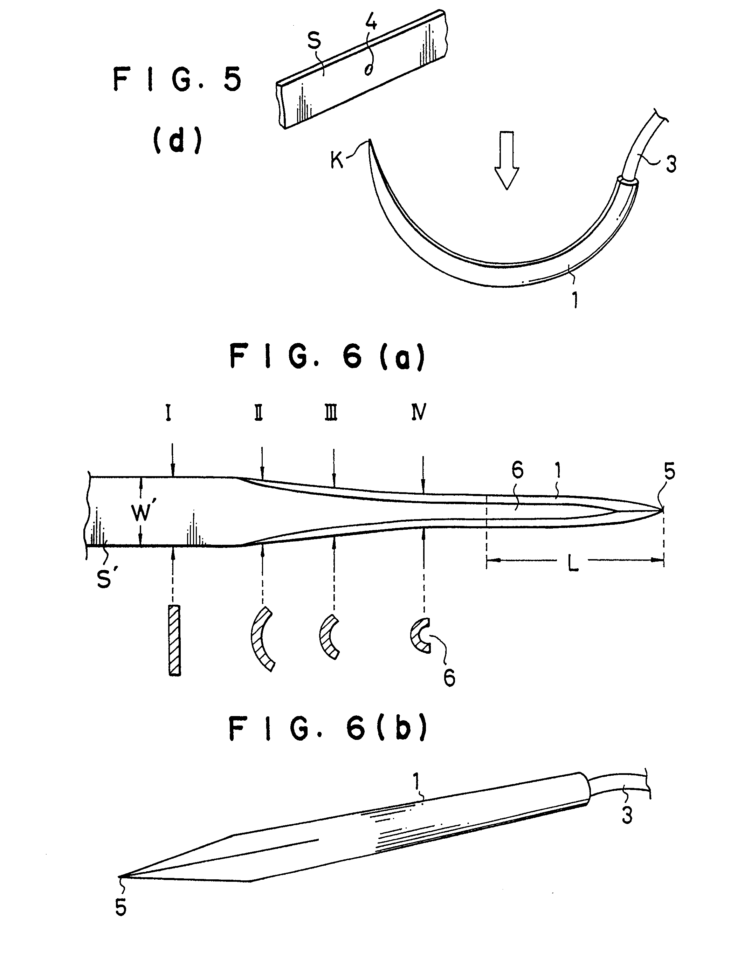

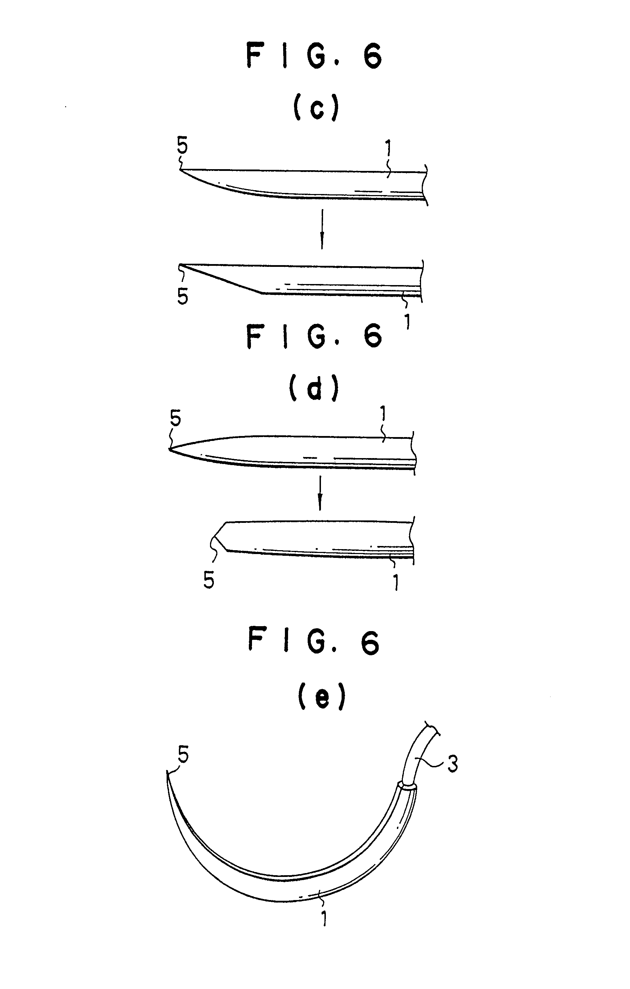

A second embodiment of the manufacturing process of the invention will be described with reference to Figs. 6(a) to 6(d).

In Fig. 6(a), there is shown an elongated stainless steel plate S' having a width W'. Such stainless steel plate is extruded in a longitudinal direction by a known forming means. The width W' is equivalent to an outer circumference of the needle body 1. The stainless steel plate S' is formed so as to obtain a semicircular groove 6 at its front part by swaging means. The semicircular groove 6 is formed to insert the suture 3 thereinto.

After a front end 5 of the stainless steel pattern S' has been formed, a length L of the front part of the stainless steel pattern S' is cut off. Thus, a grooved needle body 1 is obtained. In this way, the grooved needle body having the length L is produced one after another by the swaging means. If the front end 5 thereof is circular, it must be pointed by grinding means.

0180125

The profile of the front end 5 of the needle body 1 may be formed optionally as shown in Figs. 6(c) and 6(d). In Fig. 6(c) the front end thereof shows a sharp triangular shape by grinding, while in Fig. 6(d) it shows a pointed shape. In this way, the profile of the front end may be formed in a preferred shape by grinding means.

If a triangular-shaped end of the needle body 3 is required, preferably about one-third of the whole length of the needle body 1 is formed in a triangular shape.

Next to this step, the suture 3 is inserted into the semicircular groove 6, and firmly fixed and sealed therein by caulking both sides of the semicircular groove 6 under pressure.

In conclusion, the effects and aspects of a suture needle according to this invention can be summarized as follows.

(1) Since a suture is inserted and fixed within a needle body throughout its substantially overall length, disengagement of the suture from the needle body is entirely prevented.

(2) Since the needle body is provided with a groove, it is of a tubular structure and becomes lightweight. In addition, its resilience is enhanced.

(3) Since a suture is extended through a substantially overall length of the needle body and fixed therein, even if the needle body will locally be broken within a human body, its broken part cannot remain in it. Because the whole of the needle body is supported by the suture.

(4) Since the present invention has removed a hole structure in the prior art, cleaning and sterilization become very easy.

(5) Because of the absence of the hole structure, any cumbersome work has been removed. The products of uniform quality can be manufactured at a low cost.

Further, the effects and aspects of the processes for manufacturing a suture needle according to this invention can be summarized as follows.

(1) A large number of suture needles can be manufactured in a mass production system. Accordingly, productivity is very high.

(2) Since no manual operation is required in each production stage, quality of the products is constantly uniform. Thus, this invention can satisfy a demand for high reliability in medical instruments.

(3) Since a desired number of suture needles are formed from an elongated and flattened metal plate by means of punch press, cutting, bending or the like, a desired diameter of the needle body as well as a desired sectional profile may be predetermined optionally. In addition, the production cost is inexpensive.

While I have shown and described certain embodiments of my invention, it is to be understood that it is capable of many modifications. Changes, therefore, in the construction and arrangement may be made without departing from the spirit and scope of the invention as defined in the appended claims.

Claims:

1. A suture needle comprising a suture (3) and a needle body (1) in which a groove (1a) is extended in a substantially overall longitudinal direction of said need body, said suture being inserted into said groove and fixed therein.

2. A process for manufacturing a suture needle comprising:

forming a flattened pattern (1b) of a needle body (1) from a metal plate (S) by means of punch press;

forming a groove (1a) by bending both sides of said flattened pattern and inserting a suture (3) into said groove; and

fixing said suture in said groove by caulking both sides of said groove under pressure.

3. A process for manufacturing a suture needle as claimed in claim 2, comprising:

preparing an elongated and flattened metal plate (S) having a slightly wider width (W) than that of a needle body (1);

forming a large number of flattened and segmented patterns (1b) each being a needle body, from said elongated and flattened metal plate by means of continuous punch press;

associating a pointed end (K) of each flattened pattern integrally with a side portion of said elongated and flattened metal plate;

forming a groove (1a) by bending both sides of each flattened and segmented pattern and inserting said suture into said groove;

fixing said suture in said groove by caulking both sides of said groove under pressure and forming a needle body; and

departing said pointed end integrally associated with said side portion of said elongated and flattened metal plate from said metal body, thereby producing a finished
needle body with said suture.

4.    A process for manufacturing a suture needle as claimed
in claim 2,comprising:

transforming a metal plate (S') having a width (W')
corresponding to an outer circumference of a needle
body (1) into a semicircular tube by swaging means, thereby
forming a semicircular groove (6) in said semicircular tube
for inserting a suture (3) thereinto;

providing said semicircular tube with a pointed end
(5) by a further swaging means, and cutting off said
semicircular tube having said pointed end so as to conform
to a preferred length (L) of said needle body; and

inserting said suture wholly into said semicircular
groove and fixing it therein by caulking both sides of
said semicircular groove under pressure, thereby producing
a finished suture needle.

5.    A process for manufacturing a suture needle as
claimed in claim 4, in which an outer profile of said
pointed end of said needle body is formed in a triangular
shape by grinding means, in which case about one-third of
the whole length of the needle body 1 is preferably
formed in the triangular shape.

......................................

# F I G. 1

# F I G. 2

# F I G. 3

# F I G. 4

F I G. 5　(b)

F I G. 5
(a)

F I G. 5

(c)

FIG. 5
(d)

FIG. 6 (a)

FIG. 6 (b)

# F I G. 6
## (c)

# F I G. 6
## (d)

# F I G. 6
## (e)

# FIG. 7

## (a)

Prior Art

# FIG. 7      # FIG. 7

## (b)           ## (c)

Prior Art      Prior Art

# FIG. 7

## (d)

Prior Art